# EUROPEAN PATENT APPLICATION

(11) **EP 4 800 113 A1**
(43) Date of publication of application: **02.09.2026**
(21) Application number: 24904105.4
(22) Date of filing: 28.11.2024
(51) Int. Cl.: C12N 15/77, C12N 9/10, C12N 9/12, C12P 13/08

(54) **MICROORGANISM WITH ENHANCED ACTIVITY OF PHOSPHOTRANSACETYLASE AND ACETATE KINASE OPERON AND USES THEREOF**

(30) Priority: 14.12.2023 KR 20230181938
(71) Applicant: CJ Cheiljedang Corporation, Seoul 04560 (KR)
(72) Inventor: KIM, Seon Hye, Seoul 04560 (KR); LEE, Heeseok, Seoul 04560 (KR); YUN, Hyojin, Seoul 04560 (KR); LEE, Hyein, Seoul 04560 (KR)
(74) Representative: Meissner Bolte Partnerschaft mbB
(86) International application number: PCT/KR2024/019194
(87) International publication number: WO 2025/127540

(57) **Abstract**

The present disclosure provides a microorganism with an enhanced activity of a phosphotransacetylase and acetate kinase operon, a gene expression cassette comprising a strong promoter and a pta-ackA operon, a method for producing valine using the microorganism of the present disclosure, and a method for increasing valine production.

## Description

### [TECHNICAL FIELD]

### Cross-Reference to Related Applications

The present disclosure claims the benefit of priority to Korean Patent Application No. 10-2023-0181938, filed on December 14, 2023. The entire contents disclosed in the document of said Korean patent application are incorporated herein by reference as part of this disclosure.

The present disclosure relates to a microorganism wherein the activity of a phosphotransacetylase and acetate kinase operon is enhanced and a use thereof.

### [BACKGROUND ART]

L-amino acids, as essential building blocks of proteins, are widely used as important materials for pharmaceuticals, food additives, animal feed, nutritional supplements, pesticides, and disinfectants. Particularly, branched-chain amino acids (BCAAs) are collectively referred to as the essential amino acids of L-valine, L-leucine, and L-isoleucine, and these branched-chain amino acids are known to have antioxidant effects and an effect of directly promoting protein synthesis in muscle cells.

Meanwhile, the production of BCAAs using microorganisms is mainly carried out using microorganisms of the genus Escherichia or the genus Corynebacterium, and, in the case of L-valine, it is known to be biosynthesized from pyruvic acid via multiple steps using 2-oxoisovalerate as a precursor; however, the microbial production of L-valine is not easy for industrial-scale mass production.

Accordingly, research for effectively increasing L-valine production capacity is still needed.

### [Prior Art Documents]

### [Patent Literature]

(Patent Document 1) US Patent Publication No. US 2020-0362374 A1

### [DISCLOSURE]

### [TECHNICAL PROBLEM]

An object of the present disclosure is to provide a microorganism in which activities of a phosphotransacetylase (Pta) and acetate kinase (ackA) operon (pta-ackA operon) are enhanced.

Another object of the present disclosure is to provide a gene expression cassette comprising a promoter and a structural gene of a pta-ackA operon operably linked to the promoter.

Still another object of the present disclosure is to provide a method for producing valine, comprising culturing the microorganism in a medium.

Still another object of the present disclosure is to provide a method for increasing valine production, comprising culturing the microorganism in a medium.

Still another object of the present disclosure is to provide a composition for producing valine, comprising at least one selected from the group consisting of the microorganism and a medium in which the microorganism has been cultured.

### [TECHNICAL SOLUTION]

This can be explained in detail as follows. Meanwhile, each description and embodiment disclosed in the present disclosure may also be applied to each other description and embodiment. That is, all combinations of various elements disclosed in the present disclosure fall within the scope of the present disclosure. In addition, the scope of the present disclosure is not to be limited by the specific descriptions described below. Furthermore, a number of papers and patent documents are referenced throughout the present specification and citations thereof are indicated. The disclosures of the cited papers and patent documents are incorporated herein by reference in their entirety to more clearly explain the level of the technical field to which the present disclosure belongs and the contents of the present disclosure.

In the present disclosure, it has been found that the enhancement in the activity of a phosphotransacetylase (pta) and acetate kinase (ackA) operon (pta-ackA operon) (hereinafter, referred to as the "pta-ackA operon") of a valine-producing microorganism increases valine productivity and yield; accordingly, the present disclosure provides a promoter for enhancing the pta-ackA operon and a valine-producing microorganism in which the activity of the pta-ackA operon is enhanced.

In the present disclosure, the pta-ackA operon may comprise a structural gene of pta-ackA and a promoter operably linked thereto.

In the present disclosure, the term "promoter" refers to an untranslated polynucleotide sequence upstream of a coding region, which includes a binding site for RNA polymerase and has initiation activity for transcription of a target gene into mRNA, i.e., a DNA region that allows RNA polymerase to bind and initiate transcription of the target gene. The promoter may be located at the 5' region of the mRNA transcription initiation site.

The structural gene of the pta-ackA operon may comprise a gene (e.g., the nucleic acid sequence of SEQ ID NO: 2) encoding a pta protein (phosphotransacetylase; e.g., the amino acid sequence of SEQ ID NO: 1) and a gene (e.g., the nucleic acid sequence of SEQ ID NO: 4) encoding an ackA protein (acetate kinase; e.g., the amino acid sequence of SEQ ID NO: 3). The gene encoding the pta protein and the gene encoding the ackA protein may be linked regardless of the order. In one example, the structural gene of the pta-ackA operon may comprise the nucleic acid sequence of SEQ ID NO: 31.

The pta protein may be derived from a microorganism of the genus *Corynebacterium*, for example, from *Corynebacterium glutamicum* (Sequence ID: WP_003862872.1). The pta protein may have phosphotransacetylase activity. The sequence of the pta protein or a gene encoding the same may be obtained from a known database (NCBI) or the like. The pta protein may have homology or identity of 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more with the amino acid sequence of SEQ ID NO: 1, or may comprise the amino acid sequence, or consist of the amino acid sequence. The gene encoding the pta protein may have homology or identity of 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more with the nucleic acid sequence of SEQ ID NO: 2, or may comprise the nucleic acid sequence, or consist of the nucleic acid sequence.

The ackA protein may be derived from a microorganism of the genus Corynebacterium, for example, from *Corynebacterium glutamicum* (Sequence ID: WP_003862874.1). The ackA protein may have acetate kinase activity. The ackA protein may have phosphotransacetylase activity. The sequence of the ackA protein or a gene encoding the same can be obtained from a known database (NCBI) or the like. The ackA protein may have homology or identity of 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more with the amino acid sequence of SEQ ID NO: 3, or may comprise the amino acid sequence, or consist of the amino acid sequence. The gene encoding the ackA protein may have homology or identity of 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more with the nucleic acid sequence of SEQ ID NO: 4, or may comprise the nucleic acid sequence, or consist of the nucleic acid sequence.

In addition, as long as an amino acid sequence having such homology or identity exhibits activity corresponding to the aromatic amino acid transporter, variants having an amino acid sequence in which some portions of the sequences are deleted, modified, substituted, conservatively substituted, or added may also be included in the aromatic amino acid transporter. For example, this includes cases having sequence additions or deletions, naturally occurring mutations, silent mutations, or conservative substitutions that do not change the aromatic amino acid transporter activity at the N-terminus, C-terminus, and/or within the amino acid sequence.

The term "conservative substitution" refers to substituting one amino acid with another amino acid having similar structural and/or chemical properties. Such amino acid substitutions may generally occur based on similarities in polarity, charge, solubility, hydrophobicity, hydrophilicity, and/or amphipathic nature of the residues. Typically, the conservative substitutions may have little or no effect on the activity of a protein or polypeptide.

In the present disclosure, the expression that a polynucleotide or polypeptide "has, comprises, consists of, or essentially consists of a specific nucleic acid sequence (nucleotide sequence) or amino acid sequence" may mean that the polynucleotide or polypeptide essentially comprises the specific nucleic acid sequence or amino acid sequence, and may be interpreted as including (or not excluding mutations) a "substantially equivalent sequence" in which a mutation (deletion, substitution, modification, and/or addition) is applied to the specific nucleic acid sequence or amino acid sequence within a range that maintains the original function and/or a desired function of the polynucleotide or polypeptide. In one example, the expression that a polynucleotide or polypeptide "has, comprises, consists of, or essentially consists of a specific nucleic acid sequence (nucleotide sequence) or amino acid sequence" may mean that the polynucleotide or polypeptide (i) essentially comprises the specific nucleic acid sequence or amino acid sequence, or (ii) consists of or essentially comprises a nucleic acid sequence or amino acid sequence having 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more homology or identity with the specific nucleic acid sequence or amino acid sequence and maintains the original function and/or the desired function.

In the present disclosure, 'homology' or 'identity' refers to the degree of similarity between two given amino acid sequences or nucleotide sequences and may be expressed as a percentage. The terms homology and identity may often be used interchangeably.

The sequence homology or identity of a conserved polynucleotide or polypeptide is determined by a standard alignment algorithm, and a default gap penalty established by the program used may be used together. Substantially, homologous or identical sequences may generally hybridize with all or part of the sequence under moderate or high stringent conditions. It is obvious that hybridization also includes hybridization with a polynucleotide containing a codon considering the general codon or codon degeneracy in the polynucleotide.

Whether any two polynucleotide or polypeptide sequences have homology, similarity, or identity can be determined using a known computer algorithm such as the "FASTA" program using default parameters as in, for example, Pearson et al (1988) [Proc. Natl. Acad. Sci. USA 85]: 2444. Alternatively, it can be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later), which includes the GCG program package (Devereux, J., et al, Nucleic Acids Research 12: 387 (1984)), BLASTP, BLASTN, and FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.] (1988) SIAM J Applied Math 48: 1073). For example, homology, similarity, or identity can be determined using BLAST of the National Center for Biotechnology Information or ClustalW.

Homology, similarity, or identity of polynucleotides or polypeptides can be determined by comparing sequence information using a GAP computer program such as, for example, Needleman et al. (1970), J Mol Biol. 48:443, as known in, for example, Smith and Waterman, Adv. Appl. Math (1981) 2:482. In summary, the GAP program can be defined as the number of similarly aligned symbols (i.e., nucleotides or amino acids) divided by the total number of symbols in the shorter of the two sequences. Default parameters for the GAP program may include (1) a binary comparison matrix (containing a value of 1 for identity and 0 for non-identity) and a weighted comparison matrix as disclosed in Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for terminal gaps.

One aspect provides a microorganism in which the activity of the pta-ackA operon is enhanced. The microorganism in which the activity of the pta-ackA operon is enhanced may have one or more characteristics selected from the group consisting of (i) increased valine production capability; and (ii) increased sugar consumption rate, compared to a parent strain or a wild-type microorganism in which the activity is not enhanced.

The activity enhancement of the pta-ackA operon may mean that the activity of the operon is increased compared to its intrinsic activity. The enhancement may be used interchangeably with terms such as activation, up-regulation, overexpression, and increase. Here, activation, enhancement, up-regulation, overexpression, and increase may include showing an activity that was not originally possessed, or showing an improved activity compared to the intrinsic activity or activity before modification. The "intrinsic activity" refers to the activity of a specific operon originally possessed by the parent strain or unmodified microorganism before the transformation, in case the trait changes due to genetic variation caused by natural or artificial factors. This may be used interchangeably with "activity before modification". That the activity of an operon is "enhanced", "up-regulated", "overexpressed", or "increased" compared to the intrinsic activity may mean that the activity of a specific operon originally possessed by the parent strain or unmodified microorganism before transformation is increased, for example, that the expression of the structural gene of the operon is increased. Whether the activity of the operon is enhanced can be identified by methods commonly known in the art, such as the degree of increase in the mRNA transcription amount of the structural gene included in the operon (e.g., pta gene mRNA and/or ackA gene mRNA level), the expression levels of the polypeptide encoded by the structural gene (e.g., pta protein and/or ackA protein level), the degree of activity of the polypeptide, and the amount of product produced from the polypeptide (e.g., the production amount of valine).

Various methods well known in the art can be applied to the activity enhancement of the operon, and there is no limitation as long as the activity of the target operon can be enhanced compared to the microorganism before modification. Specifically, it may be performed by using genetic engineering and/or protein engineering well known to those skilled in the art, which are routine methods in molecular biology, but is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the activity enhancement of the pta-ackA operon may be a combination of one or more selected from 1) to 8) below, but is not limited thereto.
1) substituting the gene expression control region of the pta-ackA operon with a sequence having strong activity;
2) increasing the intracellular copy number of the pta-ackA operon;
3) modifying the nucleotide sequence encoding the initiation codon or 5'-UTR region of the structural gene of the pta-ackA operon;
4) modifying the amino acid sequence to enhance the activity of the pta protein and/or ackA protein;
5) modifying the polynucleotide sequence of the gene encoding the pta protein and/or the gene encoding the ackA protein so that the activity of the pta protein and/or ackA protein is enhanced (for example, modifying the polynucleotide sequence of the gene to encode a polypeptide modified to enhance the activity of the pta protein and/or ackA protein);
6) introducing a foreign polypeptide showing the activity of the pta protein and/or ackA protein and/or a foreign polynucleotide encoding the foreign polypeptide;
7) codon optimization of the gene encoding the pta protein and/or ackA protein; and
8) selecting an exposed site by analyzing the tertiary structure of the pta protein and/or ackA protein, and modifying or chemically modifying the exposed site.

The substitution of the gene expression control region of the pta-ackA operon with a sequence having strong activity as described in item 1) may be, for example, generating a variation in the sequence by deletion, insertion, non-conservative or conservative substitution, or a combination thereof to further enhance the activity of the expression control region, or replacement with a sequence having stronger activity. The expression control region may include a promoter, an operator sequence, a sequence encoding a ribosome binding site, and a sequence regulating the termination of transcription and translation. As an example, replacing the gene expression control region of the pta-ackA operon with a sequence having strong activity may be replacing the original promoter with a strong promoter.

The microorganism in which the activity of the pta-ackA operon is enhanced may be one in which the activity of the pta-ackA operon is enhanced by operably linking a strong promoter to the pta-ackA structural gene. That the strong promoter is operably linked to the pta-ackA structural gene as described above may refer to replacing (substituting) the promoter of the original pta-ackA operon with a strong promoter, or positioning the strong promoter in front of the pta-ackA structural gene so that the expression of the pta-ackA structural gene can be regulated by the strong promoter. The original promoter of the pta-ackA operon may mean a promoter operably linked to the pta-ackA operon in the chromosome of a wild-type microorganism. In one example, the original promoter may be a promoter linked to the pta gene.

In addition, the activity enhancement of the pta-ackA operon may include enhancing the expression of the pta gene or the ackA gene alone. In one example, the microorganism in which the activity of the pta-ackA operon is enhanced may be one in which a strong promoter is operably linked to the pta gene, and/or a strong promoter is linked to the ackA gene.

In the present disclosure, the term "operably linked" means that the promoter of the present disclosure is functionally linked with the target gene sequence (e.g., pta-ackA structural gene, pta gene, or ackA gene) so as to initiate and mediate transcription of the target gene. Operable linkage can be prepared using genetic recombination techniques known in the art of the present disclosure, and site-specific DNA cleavage and linkage can be prepared using cleavage and ligation enzymes known in the art of the present disclosure.

The strong promoter may be a promoter of an endogenous gene or a foreign gene of a microorganism. In one example, the strong promoter may be derived from a microorganism of the genus *Corynebacterium*. In one specific embodiment, the strong promoter may be derived from *Corynebacterium glutamicum*.

In one example, the strong promoter may be PctaE (promoter of a ctaE gene), Ppyk (promoter of a pyk gene), PpfkA (promoter of a pfkA gene), Pald (promoter of an ald gene), CJ1 to CJ7 promoters (U.S. Patent No. US 7662943 B2), lac promoter, trp promoter, trc promoter, tac promoter, lambda phage PR promoter, PL promoter, tet promoter, gapA promoter, SPL7 promoter, SPL13 (sm3) promoter (U.S. Patent No. US 10584338 B2), O2 promoter (U.S. Patent No. US 10273491 B2), tkt promoter, yccA promoter, etc., but is not limited thereto.

The ctaE gene may be a gene encoding cytochrome c oxidase subunit 3. In one example, the promoter of the ctaE gene may comprise the nucleic acid sequence of SEQ ID NO: 5 or consist of the nucleic acid sequence of SEQ ID NO: 5.

The pyk gene may be a gene encoding pyruvate kinase. In one example, the promoter of the pyk gene may comprise the nucleic acid sequence of SEQ ID NO: 6 or consist of the nucleic acid sequence of SEQ ID NO: 6. The pfkA gene may be a gene encoding ATP-dependent 6-phosphofructokinase isozyme 1. In one example, the promoter of the pfkA gene may comprise the nucleic acid sequence of SEQ ID NO: 7 or consist of the nucleic acid sequence of SEQ ID NO: 7.

The ald gene may be a gene encoding acetaldehyde dehydrogenase. In one example, the promoter of the ald gene may comprise the nucleic acid sequence of SEQ ID NO: 32 or consist of the nucleic acid sequence of SEQ ID NO: 32.

The increase in the intracellular copy number of the pta-ackA operon as described in item 2) may be achieved by introducing into a microorganism (host cell) a vector in which the pta-ackA operon is operably linked and which may replicate and function independently of the host. Alternatively, it may be achieved by introducing one copy or two or more copies of the pta-ackA operon into the chromosome of the microorganism (host cell). Introduction of the pta-ackA operon into the chromosome may be performed by introducing a vector capable of inserting the gene into the chromosome of the host cell into the host cell, but is not limited thereto.

The increase in the intracellular copy number of the pta-ackA operon may be achieved by introducing into a host cell a vector operably linked with the pta gene or the ackA gene, which may replicate and function independently of the host. Alternatively, it may be achieved by introducing one copy or two or more copies of the pta gene or ackA gene into the chromosome of the host cell. Introduction of the pta gene or ackA gene into the chromosome may be performed by introducing a vector capable of inserting the gene into the chromosome of the host cell into the host cell, but is not limited thereto.

The pta-ackA operon, pta gene, or ackA gene may be operably linked to a promoter.

The promoter may be at least one selected from the group consisting of the original pta-ackA operon promoter and a strong promoter that is different from the original promoter.

The original pta-ackA operon promoter and the strong promoter are as described above. The pta-ackA operon, pta gene, or ackA gene may be inserted at a position that does not affect the expression of other genes of the host cell, for example, within genomic safe harbor sites. In one example, the safe harbor gene site may be a site between the NCgl2195 gene and the NCgl2196 gene of *Corynebacterium glutamicum*, or a site between the NCgl0866 gene and the NCgl0867 gene of *Corynebacterium glutamicum*.

The modification of the nucleotide sequence encoding the start codon or 5'-UTR region of the transcript of the gene encoding the polypeptide as described in item 3) may be, for example, substitution with a nucleotide sequence encoding another start codon having a higher polypeptide expression rate than the endogenous start codon, but is not limited thereto.

The modification of the amino acid sequence or polynucleotide sequence as described in items 4) and 5) may be introducing a variation into the sequence by deletion, insertion, non-conservative or conservative substitution, or a combination thereof in the amino acid sequence of the polypeptide or the polynucleotide sequence encoding the polypeptide so as to enhance the activity of the polypeptide, or replacement with an amino acid sequence or polynucleotide sequence improved to have a stronger activity or an amino acid sequence or polynucleotide sequence improved to increase activity, but is not limited thereto. Specifically, the replacement may be performed by inserting the polynucleotide into the chromosome by homologous recombination, but is not limited thereto. The vector used therein may further include a selection marker for confirming whether it is inserted into the chromosome.

The introduction of a foreign polynucleotide exhibiting the activity of the polypeptide as described in item 6) may be the introduction of a foreign polynucleotide encoding a polypeptide exhibiting activity identical/similar to that of the polypeptide into a host cell. There is no limitation on the origin or sequence of the foreign polynucleotide as long as it exhibits activity identical/similar to the polypeptide. The method used for the introduction may be performed by a person skilled in the art appropriately selecting a known transformation method, and as the introduced polynucleotide is expressed in the host cell, a polypeptide is produced and its activity may be increased.

The codon optimization of the polynucleotide encoding the polypeptide as described in item 7) may be optimizing the codons of an endogenous polynucleotide to increase transcription or translation in a host cell, or optimizing the codons of a foreign polynucleotide so that optimized transcription and translation thereof occur in a host cell.

Analyzing the tertiary structure of a polypeptide to select and modify or chemically modify an exposed site as described in item 8) may be, for example, determining a template protein candidate according to the degree of sequence similarity by comparing sequence information of the polypeptide to be analyzed with a database in which sequence information of known proteins is stored, identifying the structure based thereon, and selecting and modifying or chemically modifying the exposed site.

Such enhancement of polypeptide activity may be an increase in the activity or expression level or concentration of the corresponding polypeptide relative to the activity or concentration of the polypeptide expressed in a wild-type or pre-modification microbial strain, or an increase in the amount of product produced from the polypeptide, but is not limited thereto.

In one specific embodiment, the enhancement may be (i) substitution of the promoter of the pta-ackA operon with a strong promoter, (ii) an increase in the copy number of the pta-ackA operon, or (iii) a combination thereof.

In the microorganism of the present disclosure, the modification of part or all of the gene for enhancing the activity of the pta-ackA operon may be induced by (a) homologous recombination using a vector for chromosome insertion in a microorganism or genome editing using an engineered nuclease (e.g., CRISPR-Cas9) and/or (b) treatment with light such as ultraviolet rays and radiation, and/or chemicals, but is not limited thereto. The method for modifying part or all of the gene may include a method by DNA recombination technology. For example, deletion of part or all of the gene may be achieved by introducing a nucleotide sequence or a vector including a nucleotide sequence having homology with a target gene into the microorganism to cause homologous recombination. The introduced nucleotide sequence or vector may include a dominant selection marker, but is not limited thereto.

The microorganism of the present disclosure may be a microorganism in which the activity of the pta-ackA operon is enhanced, or a microorganism genetically modified through a vector to enhance the activity of the pta-ackA operon (e.g., a recombinant microorganism), but is not limited thereto. The microorganism (or strain, recombinant cell) of the present disclosure may be a microorganism that has valine production capacity or has improved valine production capacity (or production amount) by enhancing the activity of the pta-ackA operon.

The microorganism of the present disclosure may be a microorganism naturally having valine production capacity, or a microorganism in which valine production capacity is conferred or enhanced in a parent strain having no valine production capacity, but is not limited thereto. The microorganism may be a microorganism having valine production capacity or having increased valine production capacity. The microorganism may be a microorganism in which valine production capacity is conferred or valine is enhanced by introducing enhancement of the pta-ackA operon into a microorganism having no valine production capacity or a microorganism having valine production capacity, but is not limited thereto.

That the microorganism has valine production capacity or has improved valine production capacity may mean that valine production capacity is conferred, unlike an unmodified microorganism without valine production capacity, a cell before recombination, a parent strain, and/or a wild-type strain, or that the valine production capacity is increased compared to an unmodified microorganism, a cell before recombination, a parent strain, and/or a wild-type strain.

A microorganism in which the activity of the pta-ackA operon is enhanced according to an embodiment, may have increased valine production capacity compared to a microorganism before introduction, i.e., an unmodified microorganism of the same species. In the present disclosure, "unmodified microorganism" does not exclude strains comprising mutations that may occur naturally in microorganisms, and may mean a wild-type strain or a natural strain itself, or a strain before traits are changed due to genetic variation caused by natural or artificial factors. For example, the unmodified microorganism may mean a microorganism in which the activity of the pta-ackA operon is not enhanced, or a microorganism before the activity of the pta-ackA operon is enhanced. The "unmodified microorganism" may be used interchangeably with "strain before modification", "microorganism before modification", "non-mutant strain", "unmodified strain", "non-mutant microorganism", or "reference microorganism".

The microorganism (or strain, recombinant cell) may additionally include a mutation that increases valine production, and the position of the mutation and/or the type of gene and/or protein to be mutated may be included without limitation as long as it increases valine production. The recombinant cell may be used without limitation as long as it is a cell capable of transformation.

In one example, the microorganism of the present disclosure may further include an A42V variant of the acetolactate synthase isozyme 1 small subunit (IlvN) protein or a nucleotide encoding the same (see Biotechnology and Bioprocess Engineering, June 2014, Volume 19, Issue 3, pp 456-467).

In one example, the unmodified microorganism, which is a strain used for comparing whether the valine production capacity is increased, may be *Corynebacterium glutamicum strain* ATCC13032, *Corynebacterium glutamicum* KCCM11201P (US 8465962 B), or a strain with improved valine production capacity by introducing an A42V mutation in the acetolactate synthase isozyme 1 small subunit (IlvN) protein in Corynebacterium glutamicum ATCC14067 [ilvN (A42V); Biotechnology and Bioprocess Engineering, June 2014, Volume 19, Issue 3, pp 456-467; US 11180784 B2], but is not limited thereto.

As an example, the microorganism (or strain, recombinant cell) with improved (increased) valine production capacity (or production amount, yield) may have increased valine production capacity by 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, 30% or more, 31% or more, 32% or more, 33% or more, 34% or more, or 35% or more (the upper limit is not particularly limited, and for example, may be about 200% or less, about 150% or less, about 100% or less, about 50% or less, about 45% or less, about 40% or less, or about 35% or less) compared to a parent strain before mutation or an unmodified microorganism, but is not limited thereto.

In another example, the microorganism (or strain, recombinant cell) with improved (increased) valine production capacity (or production amount, yield) may have increased valine production capacity by about 1.05 times or more, about 1.1 times or more, about 1.15 times or more, about 1.2 times or more, about 1.25 times or more, or about 1.3 times or more compared to a parent strain before mutation or an unmodified microorganism, but is not limited thereto. The term "about" is a range including all of ±0.5, ±0.4, ±0.3, ±0.2, ±0.1, etc., and includes all values in a range equal to or similar to the value following the term about, but is not limited thereto.

The microorganism may be a microorganism of the genus *Corynebacterium*.

The microorganism of the genus Corynebacterium may be one or more selected from the group consisting of *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium acetoacidophilum, Corynebacterium acetoglutamicum, Corynebacterium alkanolyticum, Corynebacterium lilium, Corynebacterium melassecola, Corynebacterium thermoaminogenes, Corynebacterium herculis*, and *Corynebacterium flavescens*, but is not limited thereto.

Another aspect provides a gene expression cassette including the promoter and a structural gene of a pta-ackA operon operably linked to the promoter.

In the present disclosure, the term "gene expression cassette" may mean a unit cassette that includes a promoter and a target gene and can express the target gene operably linked downstream of the promoter. Various factors capable of assisting the efficient expression of the target gene may be included inside or outside such a gene expression cassette. The gene expression cassette may typically include a transcription termination signal, a ribosome binding site, and a translation termination signal in addition to the promoter operably linked to the target gene, but is not limited thereto.

The "target gene" refers to a gene whose expression is intended to be regulated by the promoter sequence of the present disclosure for the purpose of the present disclosure. A protein encoded by the target gene may be expressed as a "target protein", and a gene encoding the "target protein" may be expressed as a "target gene".

In one example, the target gene may mean the structural gene of the pta-ackA operon to be expressed through the promoter.

The structural gene of the pta-ackA operon may include a gene encoding a pta protein (SEQ ID NO: 2) and a gene encoding an ackA protein (SEQ ID NO: 4). The structural gene of the pta-ackA operon may include the nucleic acid sequence of SEQ ID NO: 31.

The promoter may be one or more selected from the group consisting of a promoter of a ctaE gene, a promoter of a pyk gene, and a promoter of a pfkA gene described above, but is not limited thereto.

The promoter may be derived from the microorganism of the genus Corynebacterium described above, for example, from *Corynebacterium glutamicum*.

In one example, the promoter may be one or more selected from the group consisting of a promoter of the ctaE gene (SEQ ID NO: 5), a promoter of the pyk gene (SEQ ID NO: 6), and a promoter of the pfkA gene (SEQ ID NO: 7), but is not limited thereto.

In the present disclosure, the term "operably linked (operatively linked)" means that the promoter of the present disclosure is functionally linked with a target gene sequence (e.g., a structural gene of the pta-ackA operon) so as to initiate and mediate transcription of the target gene. Operable linkage can be prepared using genetic recombination techniques known in the technical field of the present disclosure, and site-specific DNA cleavage and linkage can be prepared using cleavage and linkage enzymes known in the technical field of the present disclosure.

In one example, the gene expression cassette may include at least one promoter selected from the group consisting of a promoter of a pyk gene, a promoter of a ctaE gene, and a promoter of a pfkA gene; and
a structural gene of pta-ackA operon operably linked to the promoter.

Another aspect provides a composition for valine production including at least one selected from the group consisting of the microorganism and the medium in which the microorganism is cultured. The composition of the present disclosure may further include any suitable excipients commonly used in compositions for valine production, and such excipients may be, for example, preservatives, wetting agents, dispersing agents, suspending agents, buffering agents, stabilizers, or isotonic agents, but are not limited thereto.

In the composition of the present disclosure, the microorganism (strain), medium, valine, etc., are as described in the other aspects above.

Another aspect is to provide a use for producing valine by using at least one selected from the group consisting of the microorganism, the microorganism, and the medium in which the microorganism is cultured for valine production.

Another aspect provides a method for producing valine including a step of culturing the microorganism in a medium.

Another aspect provides a method for increasing valine production including a step of culturing the microorganism in a medium.

The microorganism and valine are as described above.

In the present disclosure, "culture" may mean growing the microorganism of the present disclosure (e.g., *Corynebacterium glutamicum* strain) under appropriately controlled environmental conditions. The culturing process of the present disclosure may be carried out according to suitable media and culturing conditions known in the art. This culturing process can be easily adjusted and used by those skilled in the art according to the strain selected. Specifically, the culturing may be batch, continuous, and/or fed-batch, but is not limited thereto.

In the present disclosure, "medium" refers to a substance in which nutritional substances required for culturing the microorganism of the present disclosure (e.g., *Corynebacterium glutamicum* strain) are mixed as a main component, and supplies nutritional substances, growth factors, and the like, including water indispensable for survival and growth. Specifically, the medium and other culturing conditions used for culturing the microorganism of the present disclosure can be any without particular limitation as long as it is a medium used for culturing ordinary microorganisms, but the microorganism of the present disclosure can be cultured while adjusting temperature, pH, etc., under aerobic conditions in a common medium containing an appropriate carbon source, nitrogen source, phosphorus source, inorganic compounds, amino acids, and/or vitamins.

In one example, a culture medium for a strain of the genus Corynebacterium can be found in the literature ["Manual of Methods for General Bacteriology" by the American Society for Bacteriology (Washington D.C., USA, 1981)].

In the present disclosure, the carbon source may include carbohydrates such as glucose, saccharose, lactose, fructose, sucrose, maltose, and the like; sugar alcohols such as mannitol, sorbitol, and the like; organic acids such as pyruvic acid, lactic acid, citric acid, and the like; amino acids such as glutamic acid, methionine, lysine, and the like. In addition, natural organic nutritional sources such as starch hydrolysates, molasses, blackstrap molasses, rice bran, cassava, sugarcane bagasse, and corn steep liquor can be used, and specifically, carbohydrates such as glucose and sterilized pre-treated molasses (i.e., molasses converted to reducing sugar) and the like can be used, and other appropriate amounts of carbon sources can be used in various ways without limitation. These carbon sources may be used alone or in combination of two or more, but are not limited thereto.

As the nitrogen source, inorganic nitrogen sources such as ammonia, ammonium sulfate, ammonium chloride, ammonium acetate, ammonium phosphate, ammonium carbonate, ammonium nitrate, and the like; and organic nitrogen sources such as amino acids such as glutamic acid, methionine, glutamine, and the like, peptone, NZ-amine, meat extract, yeast extract, malt extract, corn steep liquor, casein hydrolysate, fish or decomposition products thereof, defatted soybean cake or decomposition products thereof, and the like may be used. These nitrogen sources may be used alone or in combination of two or more, but are not limited thereto.

The phosphorus source may include potassium phosphate monobasic, potassium phosphate dibasic, or sodium-containing salts corresponding thereto. As inorganic compounds, sodium chloride, calcium chloride, iron chloride, magnesium sulfate, iron sulfate, manganese sulfate, calcium carbonate, and the like may be used, and in addition, amino acids, vitamins, and/or appropriate precursors and the like may be included. These components or precursors may be added to the medium in a batch or continuous manner. However, it is not limited thereto.

In addition, during culturing of the microorganism of the present disclosure, compounds such as ammonium hydroxide, potassium hydroxide, ammonia, phosphoric acid, sulfuric acid, and the like can be added to the medium in an appropriate manner to adjust the pH of the medium. In addition, during culturing, foam generation can be suppressed by using an antifoaming agent such as fatty acid polyglycol ester. In addition, in order to maintain an aerobic state of the medium, oxygen or oxygen-containing gas may be injected into the medium, or in order to maintain anaerobic and microaerobic states, nitrogen, hydrogen, or carbon dioxide gas may be injected or no gas may be injected, but is not limited thereto.

In the culturing of the present disclosure, the culture temperature may be maintained at 20 to 45°C, specifically 25 to 40°C, and culturing may be performed for about 10 to 160 hours, but is not limited thereto.

Valine produced by the culturing of the present disclosure may be secreted into the medium or remain in the cells.

The method for producing valine or the method for increasing valine production of the present disclosure may further include a step of preparing the microorganism (strain) of the present disclosure, a step of preparing a medium for culturing the microorganism, or a combination thereof (in any order) for example, prior to the step of culturing.

The method for producing valine or the method for increasing valine production of the present disclosure may further include a step of recovering valine from the medium (medium in which culturing was performed) or the microorganism (a strain of the genus *Corynebacterium*) according to the culturing. The step of recovering may be further included after the step of culturing.

The recovery may be collecting the desired valine using a suitable method known in the art according to the culturing method of the microorganism of the present disclosure, for example, batch, continuous, or fed-batch culturing methods. For example, centrifugation, filtration, treatment with a crystallization protein precipitant (salting-out method), extraction, ultrasonic disruption, ultrafiltration, dialysis, and various chromatography (such as molecular sieve chromatography (gel filtration), adsorption chromatography, ion exchange chromatography, affinity chromatography, and the like), HPLC, or a combination of these methods may be used, and the desired valine can be recovered from the medium or microorganism using a suitable method known in the art.

In addition, the method for producing valine or the method for increasing valine production of the present disclosure may additionally include a purification step. The purification can be performed using a suitable method known in the art. In one example, when the method for producing valine or the method for increasing valine production of the present disclosure includes both a recovery step and a purification step, the recovery step and the purification step may be performed continuously or non-continuously regardless of the order, or may be performed simultaneously or integrated into one step, but are not limited thereto.

### [ADVANTAGEOUS EFFECTS]

The microorganism with enhanced activity of the phosphotransacetylase and acetate kinase operon of the present disclosure has excellent valine production capability, and thus may be efficiently utilized for the mass production of valine.

### [MODE FOR INVENTION]

### Example 1. Construction of plasmid for enhancing pta-ackA.

Based on the previously disclosed valine-producing strain Corynebacterium glutamicum KCCM11201P (U.S. Patent Publication No. US 8465962 B), a plasmid for enhancing the activity of the phosphotransacetylase and acetate kinase operon (pta-ackA operon) was constructed by performing enhancement through promoter substitution and additional gene insertion into the chromosome.

### Example 1-1. Construction of plasmid for promoter substitution.

In order to enhance the activity of the phosphotransacetylase and acetate kinase operon (pta-ackA operon), it was intended to select and enhance a promoter stronger than the expression of the endogenous pta-ackA operon, and for this purpose, a plasmid for enhancing pta-ackA activity was constructed by replacing Ppta (the wild-type promoter of the pta gene) located in front of the operon with Ppyk (the promoter of the pyk gene), PctaE (the promoter of the ctaE gene), or PpfkA (the promoter of the pfkA gene).

The amino acid sequences of phosphotransacetylase (Pta) and acetate kinase (ackA), the nucleic acid sequences of the genes encoding them, and the nucleic acid sequences of each promoter (PctaE, Ppyk, PpfkA) are as shown in Table 1 below.

Specifically, in order to construct a pta-ackA operon-enhanced strain having the PctaE, Ppyk, or PpfkA promoter, each fragment was obtained through PCR using the chromosome of the valine-producing strain *Corynebacterium glutamicum* KCCM11201P (US 8465962 B) as a template. As a polymerase for the PCR reaction, PfuUltra^{™} High-Fidelity DNA Polymerase (Stratagene) was used, and the PCR conditions were denaturation at 95°C for 30 seconds, denaturation at 55°C for 30 seconds, and polymerization at 72°C for 1 minute; and the denaturation, annealing, and polymerization steps were repeated for 28 cycles.

As a result, a 606 bp DNA fragment of the 5' upstream region of native Ppta was obtained using the primers of SEQ ID NO: 8 and SEQ ID NO: 9, and a 600 bp DNA fragment of the 3' downstream region of the native Ppta was obtained using the primers of SEQ ID NO: 10 and SEQ ID NO: 11. In addition, a 369 bp fragment of PctaE (SEQ ID NO: 5), a 490 bp fragment of Ppyk (SEQ ID NO: 6), and a 500 bp fragment of PpfkA (SEQ ID NO: 7) were respectively obtained using the primers of SEQ ID NO: 12 and SEQ ID NO: 13, SEQ ID NO: 14 and SEQ ID NO: 15, and SEQ ID NO: 16 and SEQ ID NO: 17. The primer sequences used to perform each of the PCRs using the amplified promoters and the DNA segments of the upstream and downstream regions of Ppta as templates are as shown in Table 2 below.

**[Table 1]**

| **SEQ ID NO** | **Names** | **Sequence** |
|---|---|---|
| **1** | **Pta amino acid\| sequence** | MSDTPTSALITTVNRSFDGFDLEEVAADLGVRLTNLPDEELE VSKVLAADLLAEGPALIIGVGNTFFDAQVAAALGVPVLLLVD KQGKHVALARTQVNNAGAVVAAAFTAEQEPMPDKLRKAVR NHSNLEPVMSAELFENWLLKRARAEHSHIVLPEGDDDRILM AAHQLLDQDICDITILGDPVKIKERATELGLHLNTAYLVNPLTD PRLEEFAEQFAELRKSKSVTIDEAREIMKDISYFGTMMVHN GDADGMVSGAANTTAHTIKPSFQIIKTVPEASVVSSIFLMVL RGRLWAFGDCAVNPNPTAEQLGEIAVVSAKTAAQFGIDPRV AILSYSTGNSGGGSDVDRAIDALAEARRLNPELCVDGPLQF DAAVDPGVARKKMPDSDVAGQANVFIFPDLEAGNIGYKTAQ RTGHALAVGPILQGLNKPVNDLSRGATVPDIVNTVAITAIQAG GRS |
| **2** | **Pta nucleotide sequence** | ATGTCTGACACACCGACCTCAGCTCTGATCACCACGGTC AACCGCAGCTTCGATGGATTCGATTTGGAAGAAGTAGCA GCAGACCTTGGAGTTCGGCTCACCAACCTGCCCGACGA AGAACTAGAAGTATCCAAAGTTCTCGCGGCGGACCTCCT CGCTGAGGGGCCAGCTCTCATCATCGGTGTAGGAAACAC GTTTTTCGACGCCCAGGTCGCCGCTGCCCTCGGCGTCC CAGTGCTACTGCTGGTAGACAAGCAAGGCAAGCACGTTG CTCTTGCTCGCACCCAGGTAAACAATGCCGGCGCAGTTG TTGCAGCAGCATTTACCGCTGAACAAGAGCCAATGCCGG ATAAGCTGCGCAAGGCTGTGCGCAACCACAGCAACCTC GAACCAGTCATGAGCGCCGAACTCTTTGAAAACTGGCTG CTCAAGCGCGCACGCGCAGAGCACTCCCACATTGTGCT GCCAGAAGGTGACGACGACCGCATCTTGATGGCTGCCC ACCAGCTGCTTGACCAAGACATCTGTGACATCACGATCC TTGGCGATCCAGTAAAGATCAAGGAGCGCGCTACCGAAC TTGGCCTGCACCTTAACACTGCATACCTGGTCAATCCGCT GACAGATCCTCGCCTGGAGGAATTCGCCGAACAATTCGC GGAGCTGCGCAAGTCAAAGAGCGTCACTATCGATGAAGC CCGCGAAATCATGAAGGATATTTCCTACTTCGGCACCATG ATGGTCCACAACGGCGACGCCGACGGAATGGTATCCGG TGCAGCAAACACCACCGCACACACCATTAAGCCAAGCTT CCAGATCATCAAAACTGTTCCAGAAGCATCCGTCGTTTCT TCCATCTTCCTCATGGTGCTGCGCGGGCGACTGTGGGC ATTCGGCGACTGTGCTGTTAACCCGAACCCAACTGCTGA ACAGCTTGGTGAAATCGCCGTTGTGTCAGCAAAAACTGC AGCACAATTTGGCATTGATCCTCGCGTAGCCATCTTGTCC TACTCCACTGGCAACTCCGGCGGAGGCTCAGATGTGGAT CGCGCCATCGACGCTCTTGCAGAAGCACGCCGACTCAA CCCAGAACTATGCGTCGATGGACCACTTCAGTTCGACGC CGCCGTCGACCCGGGTGTGGCGCGCAAGAAGATGCCAG ACTCTGACGTCGCTGGCCAGGCAAATGTGTTTATCTTCC CTGACCTGGAAGCCGGAAACATCGGCTACAAAACTGCAC AACGCACCGGTCACGCCCTGGCAGTTGGTCCGATTCTG CAGGGCCTGAACAAACCAGTCAACGACCTTTCCCGTGG CGCAACAGTCCCTGACATCGTCAACACAGTAGCCATCAC CGCAATTCAGGCAGGAGGACGCAGCTAA |
| **3** | **ackA amino acid sequence** | MALALVLNSGSSSIKFQLVNPENSAIDEPYVSGLVEQIGEPN GRIVLKIEGEKYTLETPIADHSEGLNLAFDLMDQHNCGPSQL EITAVGHRVVHGGILFSAPELITDEIVEMIRDLIPLAPLHNPAN VDGIDVARKILPDVPHVAVFDTGFFHSLPPAAALYAINKDVAA EHGIRRYGFHGTSHEFVSKRVVEILEKPTEDINTITFHLGNG ASMAAVQGGRAVDTSMGMTPLAGLVMGTRSGDIDPGIVFH LSRTAGMSIDEIDNLLNKKSGVKGLSGVNDFRELREMIDNN DQDAWSAYNIYIHQLRRYLGSYMVALGRVDTIVFTAGVGEN AQFVREDALAGLEMYGIEIDPERNALPNDGPRLISTDASKV KVFVIPTNEELAIARYAVKFA |
| **4** | **ackA nucleotide sequence** | ATGGCATTGGCACTTGTTTTGAACTCCGGTTCATCTTCCA TCAAATTCCAGCTGGTCAACCCCGAAAACTCTGCCATCG ACGAGCCATATGTTTCTGGTCTTGTGGAGCAGATTGGTG AGCCAAACGGCCGCATCGTACTCAAAATAGAGGGTGAAA AATACACCCTAGAGACACCCATCGCAGATCACTCCGAAG GCCTAAACCTGGCGTTCGATCTCATGGACCAGCACAACT GTGGTCCTTCCCAACTGGAAATCACCGCAGTTGGACACC GCGTGGTCCACGGTGGAATCTTGTTCTCTGCACCGGAAC TTATCACTGATGAAATCGTTGAAATGATCCGCGATCTCATT CCACTCGCACCACTGCACAACCCTGCAAACGTTGACGG CATTGATGTTGCTCGAAAAATTCTCCCCGATGTCCCACAC GTAGCTGTCTTTGACACCGGTTTCTTCCACTCACTTCCAC CAGCAGCTGCGCTGTATGCCATCAATAAAGATGTTGCCG CTGAACACGGAATCAGGCGCTATGGTTTCCACGGCACCT CTCATGAATTTGTGTCCAAGCGCGTGGTGGAAATTCTGG AAAAGCCCACCGAAGACATCAACACCATCACCTTCCACC TGGGCAACGGCGCATCCATGGCTGCTGTTCAAGGCGGC CGTGCGGTAGATACTTCCATGGGTATGACACCTCTCGCG GGACTTGTCATGGGTACCCGAAGCGGTGACATTGATCCA GGTATCGTTTTCCATCTCTCACGCACCGCTGGCATGAGC ATCGATGAGATCGATAACCTACTGAACAAGAAGTCGGGC GTTAAAGGTCTTTCCGGAGTCAATGATTTCCGTGAACTGC GGGAAATGATCGACAACAATGATCAAGATGCCTGGTCCG CGTACAACATTTACATACACCAACTCCGCCGCTACCTCGG TTCCTACATGGTGGCACTGGGACGGGTAGACACCATCGT GTTCACCGCCGGTGTCGGTGAAAATGCCCAGTTTGTCCG TGAGGATGCCTTGGCAGGTTTGGAAATGTACGGCATCGA AATCGATCCGGAGCGCAACGCACTGCCAAACGATGGTCC TCGATTGATTTCCACTGATGCCTCCAAGGTGAAGGTGTTT GTCATCCCAACTAATGAAGAATTAGCTATCGCTAGGTACG CGGTGAAGTTCGCTTAG |
| **5** | **PctaE promoter nucleotide sequence** | CATGAATCGCATTAAGCTGCAAAAACGCCTTTTATTCGGG TTCAACCAAAAGTTGTATGTGGGGCTCCAATGGGGTGAA AAACTCTGTGACGTGTCTCTCAGGCAAAAACCTGCGGAA ACCACTTTGGGCACACTCTGTGAGTTCTTCAAAATGGGA CGAGTGTGATTTGGGACAGATTGGAACTGCCTGTGAACC CTTTAACCTGCAATTATGTAACTGTCGTAAAAGGGCTATGA AGAGTTCCCGGAAGGTCGATTGAAAAGTTTGCGAATTGG GGGAAAATTCGCATCAAAAGCCGAGTTCAAACTTTCAATT GAAACGGGGGGCTTGAAGTGACTTTGGCCAACCAAACA GCCATACTAGATAGC |
| **6** | **Ppyk promoter nucleotide sequence** | CTCTACGTAGCTGGTTACACCTTGGGCCGTTTCTGGATTG AACAAATGCGCGTTGATGAAGCCACGCTTATTGGCGGCA TCCGAATCAACACCATCGTCTCCGCAGTAGTGTTTGCCG GCGCGATCATTGTGTTCTTCCTGTTGAAGAAGGGTAGGG AAACTCCCGAAGAGGTAGATCCGACTTTCACAGCGTCTG TTGCAGCAGATGCTGTAGCTTCGCCAGATGGAAAACCCT TGCCGAAAGCAGAGGAGGGCATTGATGGAGAAACGCCC TCAACGCGATAGGTTTCAACCATAGGCCTGACCTGGCTG AGATGTTTTTGGTAGAAAAACCGAGTGCCCGAATTGTTTT GTGGGTGCCCGGTTTTTCTGATTTAAGCACGTCAGAGGC GTAGAACATTGTCTGTTCACACTCTGGGTCGCAAGATTCA TCGAGAATTAATGGTAGTATCTGTGGCTTGAGGGGGAATG ACGTACTAGGCTTATGGGC |
| **7** | **PpfkA promoter nucleotide sequence** | TTCTTGGCAAGTGGGTGGGACAGGCCAAATGCACCAAC GTAGGTTGCCAGCAGGCCAGTAGCTACTGCAGGACCCTT CTTTTCATTCCAGCTTCGTGCAGCAAGCGCTCCGGATGC TGCCAATGGAATGGTGCCCAGTGGGCGAATGCCGGATTC ACGGGCAGTCAACCAACCGCCGATCAAACCTGCTGCGA CGACGGTGGCAGTGCTGACCTGGGATGCCTTTTTCAATT TCATTTCCATGGTGAGCCAGTCTAGAGACAAAATTTTTCC GCGGGGGTTTTCTTGATCTGATCCGACAACCCAATGGGG GCAAAAATGTGTCCGACCAAAAATTGTGCAGCACACCAC ATGCCCGCTCGGACAATGTCGATTTGTTAATGAAACTGCA GCTCTGGCGATTAAATAAGATGGTCAGAGACAGTTTTTTG GCCTGTCAACCCCTGTGATTCTCTTATTTTTGGGTGATTG TTCCGGCGCGGGTGTTGTGATGGGTTTAAT |

**[Table 2]**

| SEQ ID NO | Name | Sequence |
|---|---|---|
| 8 | Primer | gctcggtacccggggatccCGTCCGTGTCGGATTTCATCA |
| 9 | Primer | ctcgagACATCGCCTTTCTAGTTTCAGCC |
| 10 | Primer | ATGTCTGACACACCGACCTCAGCT |
| 11 | Primer | cctgcaggtcgactctagaAGTGTTAAGGTGCAGGCCAAG |
| 12 | Primer | AAAGGCGATGTctcgagCATGAATCGCATTAAGCTGCAAAAAC |
| 13 | Primer | TCGGTGTGTCAGACATGCTATCTAGTATGGCTGTTTGGTTG |
| 14 | Primer | AAAGGCGATGTctcgagCTCTACGTAGCTGGTTACACCTT |
| 15 | Primer | TCGGTGTGTCAGACATGCCCATAAGCCTAGTACGTCAT |
| 16 | Primer | AAAGGCGATGTctcgagTTCTTGGCAAGTGGGTGGGA |
| 17 | Primer | TCGGTGTGTCAGACATATTAAACCCATCACAACACCCGC |

PCR was performed using the amplified promoter fragments and the upstream and downstream DNA fragments of Ppta as templates and the primers of SEQ ID NO: 8 and SEQ ID NO: 11. The PCR conditions consisted of denaturation at 95°C for 5 minutes, followed by 28 cycles of denaturation at 95°C for 30 seconds; annealing at 55°C for 30 seconds; and polymerization at 72°C for 2 minutes, followed by a final polymerization reaction at 72°C for 5 minutes.

As a result, DNA fragments containing each target promoter sequence between the upstream and downstream sequences of the Ppta promoter were amplified to replace the native Ppta promoter with each promoter. The amplified products were purified using a PCR purification kit (PCR Purification kit, QUIAGEN) and used as insert DNA fragments for vector construction.

Vectors for replacing the native Ppta of *Corynebacterium glutamicum* KCCM11201P, namely, pDC24_△Pn_pta::PctaE_pta, pDC24_△Pn_pta::Ppyk_pta, and pDC24_△Pn_pta::PpfkA_pta, were constructed by cloning the amplified DNA fragments and the pDC24 vector (SEQ ID NO: 33, Table 3) treated with BamHI and XbaI (New England Biolabs, Beverly, MA) using an In-Fusion Cloning Kit from TaKaRa according to the provided manual.

**[Table 3]**

| |
|---|
| GTTCGCTTGCTGTCCATAAAACCGCCCAGTCTAGCTATCGCCATGTAAGCCCACT GCAAGCTACCTGCTTTCTCTTTGCGCTTGCGTTTTCCCTTGTCCAGATAGCCCAG TAGCTGACATTCATCCGGGGTCAGCACCGTTTCTGCGGACTGGCTTTCTACGTGT TCCGCTTCCTTTAGCAGCCCTTGCGCCCTGAGTGCTTGCGGCAGCGTGAAGCTA GCTTTTATCGCCATTCGCCATTCAGGCTGCGCAACTGTTGGGAAGGGCGATCGG TGCGGGCCTCTTCGCTATTACGCCAGCTGGCGAAAGGGGGATGTGCTGCAAGG CGATTAAGTTGGGTAACGCCAGGGTTTTCCCAGTCACGACGTTGTAAAACGACG GCCAGTGAATTCGAGCTCGGTACCCGGGGATCCTCTAGAGTCGACCTGCAGGCA TGCAAGCTTGGCGTAATCATGGTCATAGCTGTTTCCTGTGTGAAATTGTTATCCGC TCACAATTCCACACAACATACGAGCCGGAAGCATAAAGTGTAAAGCCTGGGGTGC CTAATGAGTGAGCTAACTCACATTAATTGCGTTGCGCTCACTGCCCGCTTTCCAGT CGGGAAACCTGTCGTGCCAGCTGCATTAATGAATCGGCCAACGCGCGGGGAGA GGCGGTTTGCGTATTGGGCGCTCTTCCGCTTCCTCGCTCACTGACTCGCTGCGC TCGGTCGTTCGGCTGCGGCGAGCGGTATCAGCTCACTCAAAGGCGGTAATACGG TTATCCACAGAATCAGGGGATAACGCAGGAAAGAACATGTGAGCAAAAGGCCAG CAAAAGGCCAGGAACCGTAAAAAGGCCGCGTTGCTGGCGTTTTTCCATAGGCTC CGCCCCCCTGACGAGCATCACAAAAATCGACGCTCAAGTCAGAGGTGGCGAAAC CCGACAGGACTATAAAGATACCAGGCGTTTCCCCCTGGAAGCTCCCTCGTGCGC TCTCCTGTTCCGACCCTGCCGCTTACCGGATACCTGTCCGCCTTTCTCCCTTCGG GAAGCGTGGCGCTTTCTCAATGCTCACGCTGTAGGTATCTCAGTTCGGTGTAGGT CGTTCGCTCCAAGCTGGGCTGTGTGCACGAACCCCCCGTTCAGCCCGACCGCT GCGCCTTATCCGGTAACTATCGTCTTGAGTCCAACCCGGTAAGACACGACTTATC GCCACTGGCAGCAGCCACTGGTAACAGGATTAGCAGAGCGAGGTATGTAGGCGG TGCTACAGAGTTCTTGAAGTGGTGGCCTAACTACGGCTACACTAGAAGGACAGTA TTTGGTATCTGCGCTCTGCTGAAGCCAGTTACCTTCGGAAAAAGAGTTGGTAGCT CTTGATCCGGCAAACAAACCACCGCTGGTAGCGGTGGTTTTTTTGTTTGCAAGCA GCAGATTACGCGCAGAAAAAAAGGATCTCAAGAAGATCCTTTGATCTTTTCTACGG GGTCTGACGCTCAGTGGAACGAAAACTCACGTTAAGGGATTTTGGTCATGAGATT ATCAAAAAGGATCTTCACCTAGATCCTTTTGGGGTGGGCGAAGAACTCCAGCATG AGATCCCCGCGCTGGAGGATCATCCAGCCCTGATAGAAACAGAAGCCACTGGAG CACCTCAAAAACACCATCATACACTAAATCAGTAAGTTGGCAGCATCACCCGACG CACTTTGCGCCGAATAAATACCTGTGACGGAAGATCACTTCGCAGAATAAATAAAT CCTGGTGTCCCTGTTGATACCGGGAAGCCCTGGGCCAACTTTTGGCGAAAATGA GACGTTGATCGGCACGTAAGAGGTTCCAACTTTCACCATAATGAAATAAGATCACT ACCGGGCGTATTTTTTGAGTTATCGAGATTTTCAGGAGCTGATAGAAACAGAAGC CACTGGAGCACCTCAAAAACACCATCATACACTAAATCAGTAAGTTGGCAGCATCA CCCGACGCACTTTGCGCCGAATAAATACCTGTGACGGAAGATCACTTCGCAGAAT AAATAAATCCTGGTGTCCCTGTTGATACCGGGAAGCCCTGGGCCAACTTTTGGCG AAAATGAGACGTTGATCGGCACGTAAGAGGTTCCAACTTTCACCATAATGAAATAA GATCACTACCGGGCGTATTTTTTGAGTTATCGAGATTTTCAGGAGCTCTTTGGCAT CGTCTCTCGCCTGTCCCCTCAGTTCAGTAATTTCCTGCATTTGCCTGTTTCCAGTC GGTAGATATTCCACAAAACAGCAGGGAAGCAGCGCTTTTCCGCTGCATAACCCTG CTTCGGGGTCATTATAGCGATTTTTTCGGTATATCCATCCTTTTTCGCACGATATAC AGGATTTTGCCAAAGGGTTCGTGTAGACTTTCCTTGGTGTATCCAACGGCGTCAG CCGGGCAGGATAGGTGAAGTAGGCCCACCCGCGAGCGGGTGTTCCTTCTTCACT |
| GTCCCTTATTCGCACCTGGCGGTGCTCAACGGGAATCCTGCTCTGCGAGGCTGG CCGGCTACCGCCGGCGTAACAGATGAGGGCAAGCGGATGGCTGATGAAACCAA GCCAACCAGGAAGGGCAGCCCACCTATCAAGGTGTACTGCCTTCCAGACGAACG AAGAGCGATTGAGGAAAAGGCGGCGGCGGCCGGCATGAGCCTGTCGGCCTACC TGCTGGCCGTCGGCCAGGGCTACAAAATCACGGGCGTCGTGGACTATGAGCAC GTCCGCGAGGGCGTCCCGGAAAACGATTCCGAAGCCCAACCTTTCATAGAAGGC GGCGGTGGAATCGAAATCTCGTGATGGCAGGTTGGGCGTCGCTTGGTCGGTCAT TTCGAAAAAGGTTAGGAATACGGTTAGCCATTTGCCTGCTTTTATATAGTTCATATG GGATTCACCTTTATGTTGATAAGAAATAAAAGAAAATGCCAATAGGATATCGGCATT TTCTTTTGCGTTTTTATTTGTTAACTGTTAATTGTCCTTGTTCAAGGATGCTGTCTTT GACAACAGATGTTTTCTTGCCTTTGATGTTCAGCAGGAAGCTCGGCGCAAACGTT GATTGTTTGTCTGCGTAGAATCCTCTGTTTGTCATATAGCTTGTAATCACGACATTG TTTCCTTTCGCTTGAGGTACAGCGAAGTGTGAGTAAGTAAAGGTTACATCGTTAG GCGGATCAAGATCCATTTTTAACACAAGGCCAGTTTTGTTCAGCGGCTTGTATGG GCCAGTTAAAGAATTAGAAACATAACCAAGCATGTAAATATCGTTAGACGTAATGCC GTCAATCGTCATTTTTGATCCGCGGGAGTCAGTGAACAGGTACCATTTGCCGTTC ATTTTAAAGACGTTCGCGCGTTCAATTTCATCTGTTACTGTGTTAGATGCAATCAG CGGTTTCATCACTTTTTTCAGTGTGTAATCATCGTTTAGCTCAATCATACCGAGAG CGCCGTTTGCTAACTCAGCCGTGCGTTTTTTATCGCTTTGCAGAAGTTTTTGACTT TCTTGACGGAAGAATGATGTGCTTTTGCCATAGTATGCTTTGTTAAATAAAGATTCT TCGCCTTGGTAGCCATCTTCAGTTCCAGTGTTTGCTTCAAATACTAAGTATTTGTG GCCTTTATCTTCTACGTAGTGAGGATCTCTCAGCGTATGGTTGTCGCCTGAGCTG TAGTTGCCTTCATCGATGAACTGCTGTACATTTTGATACGTTTTTCCGTCACCGTC AAAGATTGATTTATAATCCTCTACACCGTTGATGTTCAAAGAGCTGTCTGATGCTGA TACGTTAACTTGTGCAGTTGTCAGTGTTTGTTTGCCGTAATGTTTACCGGAGAAAT CAGTGTAGAATAAACGGATTTTTCCGTCAGATGTAAATGTGGCTGAACCTGACCAT TCTTGTGTTTGGTCTTTTAGGATAGAATCATTTGCATCGAATTTGTCGCTGTCTTTA AAGACGCGGCCAGCGTTTTTCCAGCTGTCAATAGAAGTTTCGCCGACTTTTTGAT AGAACATGTAAATCGATGTGTCATCCGCATTTTTAGGATCTCCGGCTAATGCAAAG ACGATGTGGTAGCCGTGATAGTTTGCGACAGTGCCGTCAGCGTTTTGTAATGGCC AGCTGTCCCAAACGTCCAGGCCTTTTGCAGAAGAGATATTTTTAATTGTGGACGA ATCAAATTCAGAAACTTGATATTTTTCATTTTTTTGCTGTTCAGGGATTTGCAGCATA TCATGGCGTGTAATATGGGAAATGCCGTATGTTTCCTTATATGGCTTTTGGTTCGTT TCTTTCGCAAACGCTTGAGTTGCGCCTCCTGCCAGCAGTGCGGTAGTAAAGGTTA ATACTGTTGCTTGTTTTGCAAACTTTTTGATGTTCATCGTTCATGTCTCCTTTTTTAT GTACTGTGTTAGCGGTCTGCTTCTTCCAGCCCTCCTGTTTGAAGATGGCAAGTTA GTTACGCACAATAAAAAAAGACCTAAAATATGTAAGGGGTGACGCCAAAGTATACA CTTTGCCCTTTACACATTTTAGGTCTTGCCTGCTTTATCAGTAACAAACCCGCGCG ATTTACTTTTCGACCTCATTCTATTAGACTCTCGTTTGGATTGCAACTGGTCTATTTT CCTCTTTTGTTTGATAGAAAATCATAAAAGGATTTGCAGACTACGGGCCTAAAGAA CTAAAAAATCTATCTGTTTCTTTTCATTCTCTGTATTTTTTATAGTTTCTGTTGCATG GGCATAAAGTTGCCTTTTTAATCACAATTCAGAAAATATCATAATATCTCATTTCACT AAATAATAGTGAACGGCAGGTATATGTGATGGGTTAAAAAGGATCACCCCAGAGTC CCGCTCAGAAGAACTCGTCAAGAAGGCGATAGAAGGCGATGCGCTGCGAATCGG GAGCGGCGATACCGTAAAGCACGAGGAAGCGGTCAGCCCATTCGCCGCCAAGC |
| TCTTCAGCAATATCACGGGTAGCCAACGCTATGTCCTGATAGCGGTCCGCCACAC CCAGCCGGCCACAGTCGATGAATCCAGAAAAGCGGCCATTTTCCACCATGATATT CGGCAAGCAGGCATCGCCATGGGTCACGACGAGATCCTCGCCGTCGGGCATCC GCGCCTTGAGCCTGGCGAACAGTTCGGCTGGCGCGAGCCCCTGATGCTCTTCG TCCAGATCATCCTGATCGACAAGACCGGCTTCCATCCGAGTACGTGCTCGCTCGA TGCGATGTTTCGCTTGGTGGTCGAATGGGCAGGTAGCCGGATCAAGCGTATGCA GCCGCCGCATTGCATCAGCCATGATGGATACTTTCTCGGCAGGAGCAAGGTGAG ATGACAGGAGATCCTGCCCCGGCACTTCGCCCAATAGCAGCCAGTCCCTTCCCG CTTCAGTGACAACGTCGAGACAGCTGCGCAAGGAACGCCCGTCGTGGCCAGCC ACGATAGCCGCGCTGCCTCGTCTTGGAGTTCATTCAGGGCACCGGACAGGTCGG TCTTGACAAAAAGAACCGGGCGCCCCTGCGCTGACAGCCGGAACACGGCGGCA TCAGAGCAGCCGATTGTCTGTTGTGCCCAGTCATAGCCGAATAGCCTCTCCACCC AAGCGGCCGGAGAACCTGCGTGCAATCCATCTTGTTCAATCATGCGAAACGATCC TCATCCTGTCTCTTGATCAGATCTTGATCCCCTGCGCCATCAGATCCTTGGCGGC AAGAAAGCCATCCAGTTTACTTTGCAGGGCTTCCCAACCTTACCAGAGGGCGCC CCAGCTGGCAATTCCG (서열번호 33) |

### Example 1-2. Construction of plasmids for gene insertion

To enhance the target gene by additionally inserting the pta-ackA operon into the chromosome of *Corynebacterium glutamicum* KCCM11201P, an intergenic site that does not code for genetic information within the KCCM11201P genome was used as an insertion site to eliminate the influence caused by gene deletion, and a non-coding region between NCg12195 and NCg12196 (hereinafter referred to as NCg12195down) was selected. At this time, a PctaE promoter, which is a promoter stronger than the native promoter or the expression of the endogenous pta-ackA operon, was utilized, and PCR was performed using the KCCM11201P genome as a template chromosome for all experiments. As a polymerase for the PCR reaction, PfuUltra^{™} High-Fidelity DNA Polymerase (Stratagene) was used, and the PCR conditions were denaturation at 95 °C for 30 seconds; denaturation at 55 °C for 30 seconds; and polymerization at 72 °C for 1 minute, and the denaturation, annealing, and polymerization steps under these conditions were repeated 28 times.

A 1200 bp fragment of the 5' upstream region of NCg12195down using the primers of SEQ ID NO: 18 and SEQ ID NO: 19, an 1157 bp fragment of the 3' downstream region of NCgl2195down using the primers of SEQ ID NO: 20 and SEQ ID NO: 21, a 3079 bp fragment of Pn_pta-ackA, which is the entire operon from the native promoter, using the primers of SEQ ID NO: 22 and SEQ ID NO: 23, a 369 bp fragment of the PctaE promoter region using the primers of SEQ ID NO: 24 and SEQ ID NO: 25, and a 2579 bp fragment of the pta-ackA operon ORF part using the primers of SEQ ID NO: 26 and SEQ ID NO: 23 were obtained, respectively. The primer sequences used to perform each of the PCRs are as shown in Table 4 below.

**[Table 4]**

| SEQ ID NO | Name | Sequence |
|---|---|---|
| 18 | Primer | gctcggtacccggggatccCGATCCATGCTGGTAGAAATCA |
| 19 | Primer | GTTCAAGTTTGCTGCCATCCCAGGT |
| 20 | Primer | TTGGCTCAAATTAGTGCCGAAGGCGA |
| 21 | Primer | cctgcaggtcgactctagaCATGCAATGGTAGACGCTCAG |
| 22 | Primer | TGGCAGCAAACTTGAACTCCTTAAAGGGGTTTTAAAAAA TGTGG |
| 23 | Primer | TCGGCACTAATTTGAGCCAACTAAGCGAACTTCACCGCG TA |
| 24 | Primer | TGGCAGCAAACTTGAACCATGAATCGCATTAAGCTGCAA AAA |
| 25 | Primer | AGGTCGGTGTGTCAGACATGCTATCTAGTATGGCTGTTTG GT |
| 26 | Primer | ATGTCTGACACACCGACCTCAGCT |

In order to prepare a gene insertion fragment using a native promoter, PCR was performed using the primers of SEQ ID NO: 18 and SEQ ID NO: 21 with the upstream and downstream fragments of NCgl2195down and the Pn_pta-ackA 3079 bp fragment, which covers from the native promoter to the entire operon, as templates, and in order to prepare a gene insertion fragment using a ctaE promoter, PCR was performed with the primers of SEQ ID NO: 18 and SEQ ID NO: 21 using the upstream and downstream fragments of NCgl2195down, the PctaE promoter fragment, and the 2579 bp fragment of the pta-ackA operon ORF region as templates. The PCR conditions consisted of denaturation at 95°C for 5 minutes, followed by 28 cycles of denaturation at 95°C for 30 seconds; annealing at 55°C for 30 seconds; and polymerization at 72°C for 2 minutes, and then a final polymerization reaction was performed at 72°C for 5 minutes.

Vectors for additional insertion of the pta-ackA operon of *Corynebacterium glutamicum* KCCM11201P, pDC24_△NCgl2195down::Pn_pta-ackA and pDC24_△NCgl2195down::PctaE_pta-ackA, were constructed by cloning the amplified DNA fragments and pDC24 vector treated with BamHI and XbaI (New England Biolabs, Beverly, MA) using an In-fusion Cloning Kit from TaKaRa according to the provided manual.

### Example 2. Construction of a strain with enhanced activity of phosphotransacetylase and acetate kinase operon (pta-ackA operon) and evaluation of L-valine production capacity

### Example 2-1. Construction of promoter-substituted strains

The pDC24_△Pn_pta::PctaE_pta, pDC24_△Pn_pta::Ppyk_pta, and pDC24_△Pn_pta::PpfkA_pta vectors constructed in Example 1-1 were transformed into *Corynebacterium glutamicum* KCCM11201P by homologous recombination on the chromosome (van der Rest et al., Appl Microbiol Biotechnol 52:541-545, 1999). Strains in which the vectors were inserted into the chromosome by recombination of homologous sequences were selected on a medium containing 25 mg/l of kanamycin. Subsequently, for the *Corynebacterium glutamicum* transformants in which secondary recombination was completed, PCR was performed using the primers of SEQ ID NO: 8 and SEQ ID NO: 11, and the nucleotide sequences were confirmed to verify whether the promoter was replaced. Strains in which the promoter of the pta gene was replaced in the parent strain KCCM11201P on the chromosome were named *Corynebacterium glutamicum* KCCM11201P_△Pn_pta::PctaE_pta, KCCM11201P_△Pn_pta::Ppyk_pta, and KCCM11201P_△Pn_pta::PpfkA_pta, respectively.

### Example 2-2. Evaluation of L-valine production capacity of promoter-substituted strains

Flask evaluation was performed to compare the valine production capacity of the valine-producing strai*n Corynebacterium glutamicum* KCCM11201P and the four types of strains constructed in Example 2-1, namely KCCM11201P_△Pn_pta::PctaE_pta, KCCM11201P_△Pn_pta::Ppyk_pta, and KCCM11201P_△Pn_pta::PpfkA_pta. After sub-culturing each strain in a nutrient medium, each strain was inoculated into a 250 mL corner-baffle flask containing 25 mL of production medium, and cultured with shaking at 200 rpm at 30°C for 72 hours. The final OD, valine production yield, and relative sugar consumption rate of each strain were measured and are shown in Table 5 below.

### [Nutrient medium (pH 7.2)]

Glucose 10 g, beef extract 5 g, polypeptone 10 g, sodium chloride 2.5 g, yeast extract 5 g, agar 20 g, urea 2 g (based on 1 liter of distilled water)

### [Production medium (pH 7.0)]

Glucose 100 g, ammonium sulfate 40 g, soybean protein 2.5 g, Corn Steep Solids 5 g, urea 3 g, potassium phosphate dibasic 1 g, magnesium sulfate heptahydrate 0.5 g, biotin 100 µg, thiamine-HCl 1 mg, calcium pantothenate 2 mg, nicotinamide 3 mg, calcium carbonate 30 g (based on 1 liter of distilled water)

**[Table 5]**

| Strain | FN OD | Valine yield | Increase in valine yield (compared to parent stain) | Sugar consumption rate (compared to parent stain) |
|---|---|---|---|---|
| | 562 nm | % | % | % |
| KCCM11201P | 71.8 | 2.7 | 100 | 100 |
| KCCM11201P_ΔPn_pta ::PctaE_pta | 70.4 | 3.5 | 129.63 | 109 |
| KCCM11201P_ΔPn_pta ::Ppyk_pta | 67.8 | 3.3 | 122.22 | 104 |
| KCCM11201P_ΔPn_pta ::PpfkA_pta | 69.9 | 3.0 | 111.11 | 104 |

As shown in Table 5, when the native promoter was substituted to enhance the expression of the pta-ackA operon, all strains were superior to the parent strain (KCCM11201P) in terms of the relative sugar consumption rate, and the yield did not decrease, thereby confirming that valine productivity was improved through the enhancement of the pta-ackA operon expression.

### Example 2-3. Construction of strains with additional gene insertion

To evaluate the effect of improving valine productivity when the expression of the pta-ackA operon is enhanced by promoter substitution and the enhancement effect through additional insertion of the pta-ackA operon into the chromosome, additional gene insertion was performed using the ctaE promoter, which was most effective in Example 2-2, and the native promoter. The pDC24_△NCgl2195down::Pn_pta-ackA and pDC24_△NCgl2195down::PctaE_pta-ackA vectors constructed in Example 1-2 were transformed into *Corynebacterium glutamicum* KCCM11201P by homologous recombination on the chromosome (van der Rest et al., Appl Microbiol Biotechnol 52:541-545, 1999). Strains in which the vectors were inserted into the chromosome by recombination of homologous sequences were selected on a medium containing 25 mg/L of kanamycin. Subsequently, the *Corynebacterium glutamicum* transformants in which the second recombination was completed were subjected to PCR using primers of SEQ ID NO: 18 and SEQ ID NO: 21, and the nucleotide sequences were confirmed, so as to identify the promoter substitution. The strains in which the pta-ackA operon was additionally inserted in the parent strain KCCM11201P on the chromosome were named *Corynebacterium glutamicum* KCCM11201P_△NCgl2195down::Pn_pta-ackA and KCCM11201P_△NCgl2195down::PctaE_pta-ackA, respectively.

### Example 2-4. Evaluation of L-valine production capacity of strains with additional gene insertion

To compare the valine production capacities of the valine-producing strai*n Corynebacterium glutamicum* KCCM11201P and the two strains constructed in Example 2-3, KCCM11201P_△NCgl2195down::Pn_pta-ackA and KCCM11201P_△NCgl2195down::PctaE_pta-ackA, the flask evaluation was performed as in Example 2-2. The final OD, valine production yield, and relative sugar consumption rate of each strain were measured and are shown in Table 6 below.

**[Table 6]**

| Strain | FN OD | Valine yield | Increase in valine yield (compared to parent strain) | Relative sugar consumption rate (compared to parent strain) |
|---|---|---|---|---|
| | 562 nm | % | % | % |
| KCCM11201P | 71.9 | 2.7 | 100 | 100 |
| KCCM11201P_ΔNCgl2 195down::Pn_pta-ackA | 72.2 | 2.8 | 103.70 | 102 |
| KCCM11201P_ΔNCgl2 195down::PctaE_pta-ackA | 69.7 | 3.3 | 122.22 | 107 |

As shown in Table 6, as a result of additionally inserting the pta-ackA gene to enhance the expression of the pta-ackA operon, it was confirmed that the relative sugar consumption rate and the valine yield increased compared to those of the parent strain (KCCM11201P).

### Example 2-5. Evaluation of valine production capacity of Corynebacterium glutamicum CJ7V strain

In order to evaluate whether the effect of enhancing the pta-ackA operon increases the valine production capacity in other strains belonging to *Corynebacterium glutamicum* that produce valine, a strain with improved valine production capacity was prepared by introducing a mutation [ilvN (A42V); Biotechnology and Bioprocess Engineering, June 2014, Volume 19, Issue 3, pp 456-467] into the acetolactate synthase isozyme 1 small subunit (IlvN) protein of wild-type *Corynebacterium glutamicum* ATCC14067.

Specifically, to construct a vector for introducing A42V mutation into the ilvN gene, the genomic DNA of wild-type *Corynebacterium glutamicum* ATCC14067 strain was extracted using a G-spin Total DNA extraction mini kit (Intron Co., Cat. No 17045) according to the manufacturer's protocol. PCR was performed using the genomic DNA as a template and primer pairs of SEQ ID NO: 27 and SEQ ID NO: 28 and primer pairs of SEQ ID NO: 29 and SEQ ID NO: 30 to obtain gene fragments A and B, respectively. PCR conditions consisted of denaturation at 94 °C for 5 minutes; followed by 28 cycles of denaturation at 94 °C for 30 seconds, annealing at 55 °C for 30 seconds, and polymerization at 72 °C for 60 seconds; and a final polymerization at 72 °C for 7 minutes. As a result, gene fragment A of 528 bp and gene fragment B of 509 bp were obtained. Overlapping PCR was performed using the obtained gene fragments A and B as templates and the primer pair of SEQ ID NO: 27 and SEQ ID NO: 30. As a result, a 1010 bp PCR product (hereinafter referred to as "mutation-introduced fragment 2") was obtained.

The mutation-introduced fragment 2 obtained above was treated with the restriction enzyme SmaI and then ligated with the pDC24 vector treated with the same restriction enzyme, which was then transformed into *Escherichia coli* DH5α strain (INVITROGEN, DH5a competent cell) by electroporation to induce homologous recombination on the chromosome. Strains in which the vector was inserted into the chromosome by recombination of homologous sequences were selected on LB medium containing kanamycin. DNA was obtained from the selected *E. coli* transformants using a DNA-spin plasmid DNA purification kit according to the manufacturer's protocol, and a pDC24-ilvN(A42V) vector for introducing the A42V mutation into the ilvN gene, including mutation-introduced fragment 2, was constructed.

The primer sequences used herein are shown in Table 7 below.

**[Table 7]**

| SEQ ID NO | Name | Sequence(5' -> 3') |
|---|---|---|
| 27 | primer | cggggatcccccgggAGGACGGTACTCAAATACTAAACTT C |
| 28 | primer | TGCCGAGTGTTTCGGTCTTTACAGACACGAGGGAC ACG |
| 29 | primer | TGTCTGTAAAGACCGAAACACTCGGCATCAA |
| 30 | primer | cggggatcccccgggGACAACTACATTATTATTATACCACA |

The pDC24-ilvN(A42V) vector prepared above was transformed into wild-type *Corynebacterium glutamicum* ATCC14067 by homologous recombination on the chromosome. Strains in which the vector was inserted into the chromosome by recombination of homologous sequences were selected on a medium containing 25 mg/L of kanamycin. Thereafter, PCR using a primer pair of SEQ ID NO: 27 and SEQ ID NO: 30 was performed on the *Corynebacterium glutamicum* transformants in which secondary recombination was completed to amplify a gene fragment, and then a strain in which the A42V mutation was introduced into the ilvN gene was identified through gene sequence analysis. The recombinant strain was named *Corynebacterium glutamicum* CJ7V.

Finally, the pDC24-△Pn_pta::PctaE_pta and pDC24-△NCgl2195down::PctaE_pta-ackA vectors were transformed into the *Corynebacterium glutamicum* CJ7V in the same manner as in Examples 2-1 and 2-3. The recombinant strains were named *Corynebacterium glutamicum* CJ7V-△Pn_pta::PctaE_pta and CJ7V-△NCgl2195down::PctaE_pta-ackA.

The L-valine production capacities of the parent strain CJ7V and the CJ7V-△Pn_pta::PctaE_pta and CJ7V-△NCgl2195down::PctaE_pta-ackA strains were evaluated in the same manner as in Example 2-2 and are shown in Table 8 below.

**[Table 8]**

| Strain | FN OD | Valine yield | Increase in valine yield (compared to parent strain) | Relative sugar consumption rate (compared to parent strain) |
|---|---|---|---|---|
| | 562 nm | % | % | % |
| CJ7V | 137.3 | 2.2 | 100 | 100 |
| CJ7V-ΔPn_pta::PctaE_pta | 123.9 | 2.7 | 122.73 | 108 |
| CJ7V-ΔNCgl2195down::PctaE_pt a-ackA | 128.4 | 2.6 | 118.18 | 106 |

As a result, it was reconfirmed that the sugar consumption rate and the valine yield increased when the pta-ackA operon of *Corynebacterium glutamicum* producing valine was enhanced.

From the foregoing description, those skilled in the art to which the present disclosure pertains will be able to understand that the present disclosure may be embodied in other specific forms without changing its technical spirit or essential characteristics. In this regard, it should be understood that the embodiments described above are illustrative in all respects and not restrictive. The scope of the present disclosure should be interpreted such that all modifications or variations derived from the meaning and scope of the claims to be described later and their equivalent concepts, rather than the detailed description above, are included in the scope of the present disclosure.

## Claims

1. A microorganism wherein activity of a phosphotransacetylase and acetate kinase operon (pta-ackA operon) is enhanced.

2. The microorganism according to claim 1, wherein the enhancement is
(i) substitution of a promoter of the pta-ackA operon with a strong promoter;
(ii) an increase in the copy number of the pta-ackA operon; or
(iii) a combination thereof.

3. The microorganism according to claim 2, wherein the strong promoter is at least one selected from the group consisting of a promoter of a pyk gene, a promoter of a ctaE gene, and a promoter of a pfkA gene.

4. The microorganism according to claim 2, wherein the promoter of the pta-ackA operon is substituted with a promoter comprising at least one nucleic acid sequence selected from the group consisting of SEQ ID NO: 5, SEQ ID NO: 6, and SEQ ID NO: 7.

5. The microorganism according to claim 1, wherein the pta-ackA operon comprises the nucleic acid sequence of SEQ ID NO: 2 and the nucleic acid sequence of SEQ ID NO: 4.

6. The microorganism according to claim 1, wherein valine production capacity is increased.

7. The microorganism according to claim 1, wherein the microorganism is a microorganism of the genus *Corynebacterium*.

8. The microorganism according to claim 7, wherein the microorganism of the genus *Corynebacterium* is *Corynebacterium glutamicum*.

9. A gene expression cassette comprising:
(a) at least one promoter selected from the group consisting of a promoter of a pyk gene, a promoter of a ctaE gene, and a promoter of a pfkA gene; and
(b) a structural gene of pta-ackA operon operably linked to the promoter.

10. A method for producing valine, comprising a step of culturing the microorganism of claim 1 in a medium.

11. The method according to claim 10, further comprising a step of recovering valine from the medium or the microorganism following the culturing.

12. A method for increasing valine production, comprising a step of culturing the microorganism of claim 1 in a medium.
